# EUROPEAN PATENT APPLICATION

(11) **EP 4 300 507 A1**
(43) Date of publication of application: **03.01.2024**
(21) Application number: 22181629.1
(22) Date of filing: 28.06.2022
(51) Int. Cl.: G16H 15/00, G16H 10/60

(54) **AN AUTOMATED SYSTEM AND METHOD FOR MANAGING AN ARTIFICIAL INTELLIGENCE HEALTH PLATFORM**

(71) Applicant: el Bakri, Charles Adnan, 51500 Villers-Allerand Reims (FR)
(72) Inventor: el Bakri, Charles Adnan, 51500 Villers-Allerand Reims (FR)
(74) Representative: Fidal Innovation

(57) **Abstract**

An automated system (1) for managing an artificial intelligence health platform comprising:
➢ a first data storage system (11) storing
o a plurality of user identification elements, each user identification element being associated with a user among a plurality of users,
o for each user, at least one health document linked to said user identification element,

➢ a server (10) configured to communicate with said first data storage system (11), said server being configured to:
o receive a message with an attached file, said attached file being an attached health document,
o implement an artificial intelligence module (101) implementing a machine learning model, said machine learning model being configured to perform optical recognition and data extraction in the attached health document, said extracted data being a user identification element,

record and link said health document to the corresponding user in the first data storage system (11) on the basis of the extracted personal identification element

## Description

### FIELD OF THE INVENTION

The present invention relates to the field of automated system for managing an artificial intelligence health platform and an automated method for managing an artificial intelligence health platform.

### BACKGROUND OF THE INVENTION

In the field of health platform, document US2016/0232296 describes the management of a health file. The health file is accessible for a plurality of patients through an application, where each of the patients have a personal profile. The patients are able to file health documents in their profile. The health documents are scanned by an optical character recognition server so the sentence in the health document is transformed into a plain text which is then posted on the user's profile.

One drawback of this document is that the management is not entirely automated. The user needs to connect itself to its profile and upload a health document, which is time consuming. Another drawback of this document is that, since the user is the only one able to charge a document on its profile, he needs to require a copy of any medical exam or doctor prescription when leaving the appointment of the doctor, which does not result with a health platform facilitating the everyday life of a patient using the health platform.

The present invention aims to answer at least partially to the above exposed problems.

### BRIEF SUMMARY OF THE INVENTION

To this aim, the present application relates to an automated system for managing an artificial intelligence health platform comprising:
➢ a first data storage system storing
   ∘ a plurality of user identification elements, each user identification element being associated with a user among a plurality of users,
   ∘ for each user, at least one health document linked to said user identification element,
➢ a server configured to communicate with said first data storage system, said server being configured to:
   ∘ receive a message with an attached file, said attached file being an attached health document,
   ∘ implement an artificial intelligence module implementing a machine learning model, said machine learning model being configured to perform optical recognition and data extraction in the attached health document, said extracted data being a user identification element,
   ∘ record and link said health document to the corresponding user in the first data storage system on the basis of the extracted personal identification element.

The message may be an email, an sms (i.e. a text massage), a mms, or a message via an electronic messaging service from a dedicated application (i.e. by a messaging service such as Whatsapp, Messenger, Telegram, etc.). In the case where it is an email, the email address is unique and common for all users. .). In the case where it is a sms or a mms, the phone number to which it is sent is unique and common for all users. In the case where it is via a messaging service, the profile to which it is sent is unique and common for all users. A message can be sent by any person, without being a patient or a user of the health platform.

The system according to the invention improves the automation of the health platform since the machine learning model is able to analyse and classify any type of documents, without the need for a particular user to connect to their profile on the user interface and upload the health document. As an example, after a doctor appointment, the user does not need to request for a hard copy (a paper copy) of any document delivered by the doctor to put it on its profile. Instead, the doctor itself can simply send the document to the server, without the need to identify the patient. The document will then be uploaded automatically on the user's profile

According to different aspects, it is possible to provide the one and / or the other of the characteristics below taken alone or in combination.

According to one embodiment, the system further comprises a user terminal comprising a user interface, said user terminal being configured to communicate with the server, wherein the user terminal is configured to allow a user to visualize their health document linked to their user personal identification element.

According to one embodiment, following the recording and linking of said health document to the corresponding user in the first data storage system, the server is further configured to send a notification to the corresponding user, said notification indicating that a health document is accessible via the user's interface.

This embodiment allows the user to keep track of the updating of its profile with new health document.

According to an embodiment, the notification is sent to the user terminal, and/or to a phone number of the user and/or to an email address of the user.

Multiple kinds of notification may then be sent to the user, ensuring that the user is aware immediately that a new health document has been uploaded on their profile. In an embodiment, the user may choose which kind of notification they wish to receive.

According to an embodiment, upon the receiving of the notification, the user may confirm to the server whether the recorded and linked health document has been linked and recorded to the correct user.

According to this embodiment, if there has been a mistake by the machine learning model and/or the server according to the recording and linking of the health document, the error can be corrected immediately. Such error may happen if several users possess the same personal identification element (for example if the personal identification element is a name, several users may have the same name).

According to an embodiment, the machine learning model of the artificial intelligence module is further configured to classify the attached health data document into one of the following classes of document text document, image document such as X-Ray imaging, echography, photography document of a part of a body.

The artificial intelligence is then further trained to classify a document into several types of documents. This also improves the automation of the management of the health platform since a user does not have to open the document and determine what is it about before classifying it in a particular class of documents. It then participates to reduce any interaction of the user with the health platform.

According to an embodiment, the system further comprises a second data storage system in communication with said server, said second data storage system storing at least drugs names and diseases names, said second data storage system being automatically and continuously constructed by means of the machine learning model of the artificial intelligence module, wherein said machine learning model is configured to scan at least one existing official database regrouping drug names and diseases name and to associate said drug names and disease names with one single identifier.

This embodiment allows to render the management of the health platform even more autonomous since the system comprises its own database regrouping every official names of drugs, diseases, allergies, vaccines and biological parameters. The health platform then does not rely on any other systems since the second database is a part of the system. Furthermore, since the official databases are common all around the world, the health platform may be used in every country of the world very easily.

The second data storage system may also comprise allergies names, vaccines names and biological parameters names.

According to an embodiment, said machine learning model of the artificial intelligence module is further configured to regularly update said second database with newly added drug names and disease names identified in the official database.

This improves the accuracy of the data stored in the second database.

According to an embodiment, the machine learning model of the artificial intelligence module is further configured to classify sentences from the documents classified in the text document class between useful sentences and useless sentences, the artificial intelligence module being further configured to attach said useful sentences to one single identifier of the second database.

A useful sentence comprises a drug name, a diagnostic including a diagnostic of a disease, a posology. When the second data storage system comprises allergies vaccines, and biological parameters, the useful sentences comprise a biological parameter, a vaccine.

This embodiment also improves the automation of the management of the health platform since the association of a particular sentence with its single identifier is made by the artificial intelligence, without any need for a human intervention. Moreover, it allows to have a unified classification, such that the health platform can be used everywhere in the world without the need for a user to find the equivalent of a useful sentence in its country, which could be something else elsewhere.

For example, a lot of drugs using same chemical components are used in the world with different names. One can cite for example the drug called "doliprane" in some countries in the world but not in other countries. Doliprane uses the chemical component "paracetamol" which is called in the International Common Denomination database "acetaminophen". The second database may then comprise a single identifier for all the drugs using paracetamol, such that whatever the drug is called in a country, every doctor in the world may know that this particular drug actually uses acetaminophen

According to an embodiment, the user terminal comprises a user interface that allows the user to access the health platform, said user interface being a website, a web application or a smartphone application.

The health platform is thus accessible through multiple terminals.

According to an embodiment, the attached document is a digital document in one of the following formats: pdf and/or image format.

The machine learning model is then able to scan and analyse multiple sorts of formats documents.

According to an embodiment, the user identification element comprises a name and/or a forename of said user, an insurance number, and/or a personal code linked to their profile.

The use of multiple user personal identification elements may help reduce the risk of linking a health document to the wrong user.

The present application also related to an automated method for managing an artificial intelligence health platform managing system comprising:
> storing in a first data storage system:
   ∘ a plurality of user identification elements, each user identification element being associated with a user among a plurality of users,
   ∘ for each user, at least one health document linked to said user identification element,
> providing a communication between a server and said first data storage system, wherein said server:
   ∘ receives an message with an attached file, said attached file being an attached health document,
   ∘ implements an artificial intelligence module implementing a machine learning model performing optical recognition data extraction in the attached health document, said extracted data being a user personal identification element,
   ∘ records and links said health document to the corresponding user in the data storage system on the basis of the extracted user identification element.

### BRIEF DESCRIPTION OF THE DRAWINGS

Embodiments of the invention will be described below with reference to the drawings, described briefly below:
[Fig. 1] represents an example of a profile of a user on the health platform according to an embodiment,
[Fig. 2] represents a system for managing a health platform according to an embodiment,
[Fig. 3] represents some steps of a method for managing a health platform according to an embodiment,
[Fig. 4] represents some steps of the implementation of an artificial intelligence module according to an embodiment.

In the drawings, identical references designate identical or similar objects.

### DETAILED DESCRIPTION OF THE INVENTION

Succinctly, the health platform according to the application, is a platform where users can access their medical file, i.e., their health documents and health data. The health platform must answer to several constraints such as the respect of the privacy and a secured exchange of data, especially in the health field. In the present application, the health platform is accessible to a plurality of users through a dedicated profile. In other words, each user of the health platform has their own personal profile, through each they can access their health data and document.

A user may obtain a profile by registering for the service. In an embodiment, after registering the user is granted with a card linked to a single and unique key code. With this key code, the user can create its profile on the health platform.

A user may be a patient or a healthcare professional.

In an embodiment, all users have their personal profile, where they can access their health data and health documents, register their user identification elements such as their name and forename and/or their insurance number. The personal key code may also be considered as a user identification element.

Concerning the users being patients, besides their health file they may have access to list of healthcare professionals using the health platform.

In an embodiment, the healthcare professional may have a profile corresponding to a patient profile, but also a professional profile. On their professional profile, a healthcare professional may be able to visualize all the profiles of their patients.

According to an embodiment, a patient of a healthcare professional may be able to choose whether or not to share their profile with their healthcare professional or which specific health data or health document they wish to share with their healthcare professional. They may choose to share only some of the health data and/or health documents present on their profile.

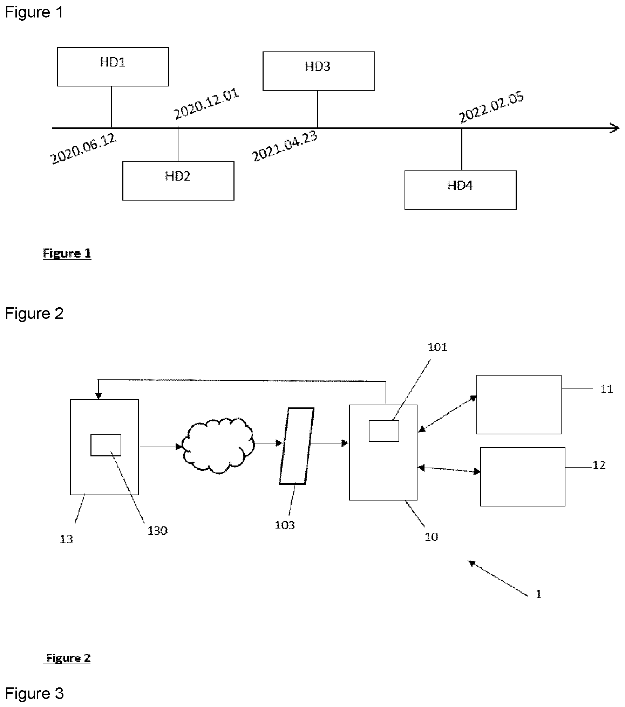

When a user, and more particularly when a patient, accesses their profile on the health platform, their health data and health documents may be displayed according to various presentation. Figure 1 gives an example of such presentation, where the health data and health documents HD1, ..., HD4 are displayed according to a timeline. Time is represented by the arrow and the blocks HD1, ... HD4 correspond to their health document and/or health data, associated with a date.

Regarding what a patient chooses to disclose to their healthcare professional, the professional may also be able to visualize their timeline displaying their health documents and health data.

Furthermore, each user (patient or professional) may be able to communicate documents or just talk to each other by means of a chat and/or videocalls implemented by an interactive telecommunication tool accessible for the users via their profile.

It is also possible for the users to navigate in their profile, select filters to apply in a search-bar accessible in their profile. For instance, the user can choose to see only the health document and/or health data between two dates, or only a certain type of health document.

The health data may be a doctor appointment, a treatment, a fracture, etc.

The health documents may be of different types comprising a prescription, a biological test result, a hospital report, an X-Ray imaging and/or a photography of a body part, such as a photography of a scar.

Globally, a management system 1 according to an embodiment is illustrated on figure 2.

The system 1 comprises a server 10, a first data storage system 11, a second data storage system 12 and at least one user terminal 13.

In an embodiment, the server 10 and the data storage system 11 are configured to communicate with one another, by exchanging data. The communication may be made through a network, as internet.

The first data storage system 11 may store, among other things, a plurality of user identification elements, each user identification element being associated with a user among the plurality of users using the health platform. The first data storage system 11 also stores, for each user, at least one health document linked to said user identification element. The first data storage system 11 may be a database storing the above-mentioned data.

When a user is connecting to its profile via the user terminal 13, it may send a request to the server 10 that retrieves the health document associated with the user identification element in the first data storage system 11.

In an embodiment, the server 10 and the second data storage system 12 are configured to communicate with one another, by exchanging data. The communication may be made through a network, as internet.

The second data storage system 12 may store a list of drugs, diseases names, each of the names being linked to a single identifier. The second data storage system 12 may be a database storing the above-mentioned data. The second data storage system 12 will be described in more details below.

In an embodiment, the server 10 and the user terminal 13 are configured to communicate with one another by exchanging data. The communication may be made through a network, as internet.

To protect the data exchanged between the server 10 and the user terminal 13, a firewall 103 may be provided, that would help respect the security and the privacy during the communication.

The user terminal 13 may be a computing device such as a computer, a smartphone or a tablet. The system 1 also comprises a user interface 130 that can be accessible through the user terminal 13. The user interface 130 may for example be a website, a desktop application or a smartphone or tablet application.

In an embodiment, the server 10 implements an artificial intelligence module 101. 101

The artificial intelligence module 101 may comprise at least one machine learning model trained to perform optical recognition and data extraction in health documents, as will be described in more details below.

In a preferred embodiment, the machine learning model is trained in a multitask way to perform optical recognition and data extraction in health documents.

The training of the machine learning model in a multitask way enables the machine learning model to perform both of the optical recognition and data extraction at the same time.

The machine learning model of the artificial intelligence module 101 may further be trained to automatically and continuously construct the second data storage system 12.

More precisely, the machine learning model of the artificial intelligence module 101 is trained to scan at least one existing database regrouping drug names and diseases names. Such existing database is preferably an official database, accessible to public. Preferably, such existing database is a database made by the World Health Organisation or other official worldwide organisations. Such existing database generally regroups every official name of drugs or diseases.

In an embodiment, several existing official databases may be scanned. One can cite the Master Inherited Metabolic Diseases Database (IMD database).

The machine learning model of the artificial intelligence module 101 may scan the existing database and construct the second data storage system 12 with said official names of drugs and diseases. The machine learning model also associates each name of drugs and disease with a single identifier.

The health platform then lays on unified appellations. The health platform may then be used and implemented worldwide. The interoperability between different users, patients or healthcare professionals, may also be enhanced.

Indeed, a lot of drugs using same chemical components are used under different names in the world. One can cite for example the drug called "doliprane" in some countries in the world but not in other countries. Doliprane uses the chemical component "paracetamol" which is called in the International Common Denomination database "acetaminophen". The second data storage system 12 may then comprise a single identifier for all the drugs using paracetamol, such that whatever the drug is called in a country, every doctor in the world may know that this particular drug actually uses acetaminophen.

Furthermore, the machine learning model of the artificial intelligence module is trained to regularly update said second database with newly added drug names and disease names identified in the official database. This allows to add almost in real time any new name of disease or drug in the second data storage system 12. Moreover, this improves the accuracy of the data stored in the second data storage system 12.

In an embodiment, the second data storage system 12 may also comprise allergies names, vaccines names, biological parameters names.

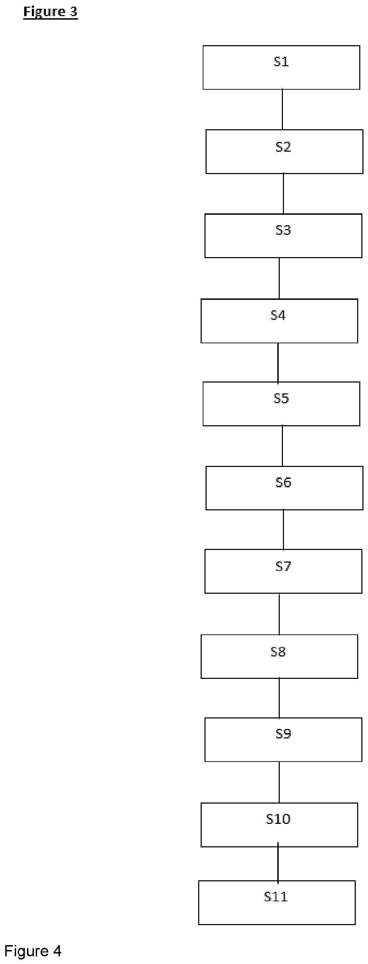

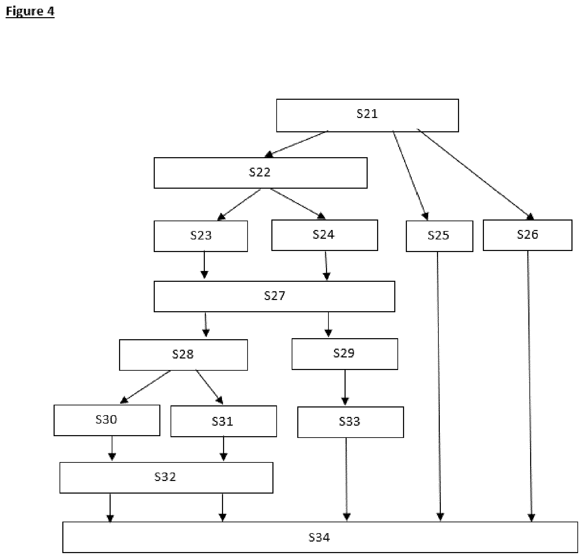

Figure 4 illustrates some steps of a management of the system 1.

At step S1, the server 10 receives a message comprising an attached file. The attached file corresponds to a health document. The message may be an email, a sms, a mms or a message sent via a messaging service such as Whatsapp, Facebook, Telegram, etc. In the case where it is an email, the email address is unique and common for all users. In the case where it is a sms or a mms, the phone number to which it is sent is unique and common for all users. In the case where it is via a messaging service, the profile to which it is sent is unique and common for all users. The message can be sent by any person, without being a patient or a user of the health platform.

For instance, after a doctor's appointment, a user of the health platform may ask the doctor to directly send all the health documents generated during the appointment directly to the dedicated phone number, email address and/or profile on the messaging service, even if the doctor is not a healthcare professional using the health platform.

Usually, after a doctor's or hospital's appointment, the patient needs to request a hard copy of any health documents generated during the appointment, either to scan it and conserve it on a computer (or on a health platform), or to conserve it as a paper in a folder, which is tedious and time consuming. In contrary, step S1 allows the patient to avoid any of these steps.

The patient may simply ask the doctor to send all the document by message.

In an embodiment, the body of the message may be empty, i.e., with no content and no object, but may solely comprise an attachment file being an attached health document.

The attached health document may be, as described above, a prescription, a biological examination result, a hospital report, an X-Ray imaging and/or a photography of a body part, such as a photography of a scar.

The attached document may be in any suitable format, including, but not limited to: pdf format, word format, image format (for example png or jpeg format). For example, a prescription may be a prescription typed on a computer which may be directly send or printed and then scanned or photographed, or written by hand and then scanned or photographed then sent. The biological examination result may also be a typed by computer document, which may be directly send or printed and then scanned or photographed then sent. The X-ray images may be also directly sent or printed and then scanned or photographed then sent. The photography of a body part may also be directly sent or printed and then scanned or photographed and sent.

At step S2, the artificial intelligence module implements the machine learning model to analyse the attached document. More precisely, the machine learning model implements an optical character recognition technique to the attached document to extract data corresponding to a user identification element. The user identification element may be a name and/or forename, an insurance number, the keycode of the user, etc.

Once the data has been extracted, the artificial intelligence module implements a classifier, as a machine learning model, to perform a classification of a document at step S3. More precisely, the classifier may be a convolutional neural network trained to classify health documents according to the extracted personal identification element. More precisely, the convolutional neural network (CNN) is able to determine to which user the health document is associated with.

At step S4, the CNN also classify the health document into one of the following classes of document: prescription class, biological examination result class, X-ray image class and photography of a body part class. For the X-ray image health document and photography of a body part health document, the method goes directly to step S7.

At step S5 the artificial intelligence module implements again the optical character recognition technique to extract text from the health documents classified in the prescription and biological examination classes.

At step S6, the CNN of the artificial intelligence module classifies sentences of the health document corresponding to a prescription and/or a biological examination result between useful sentences and useless sentences. Such useful sentences may comprise a drug name, a diagnostic including a diagnostic of a disease or an allergy, a posology, a biological parameter from a biological examination result, a vaccine. The useless sentences comprise all the other sentences.

At step S7, the artificial intelligence module associates the useful sentences with the corresponding single identifier in the second data storage system.

In an embodiment, the artificial intelligence module may even replace the useful sentence with the single identifier, as explained above for the "doliprane" drug.

At step S8, the artificial intelligence module records and links said attached health document to the corresponding user in the first data storage system, on the basis of the personal identification element extracted at step S2. Furthermore, for the corresponding user, the artificial intelligence module records and links said attached health document in a category corresponding to the class of the health document determined at step S4.

As stated above, the machine learning model is trained in a multitask way.

At step S9, a notification is sent to the corresponding user, informing them that a new health document has been added to their profile. Such notification may be sent by message, on their phone, for example as a text message (SMS) or on a specific application, and/or directly on their profile.

At step S10, the health document is displayed on the user profile. For the prescription and biological examination result class, besides the displaying of the health document, the useful sentences and/or the single identifier replacing the useful sentences may also be displayed in a structured text format.

At step S11, the user may be able to inform the server if the added health document is effectively corresponding to one of their health documents, and if no, the server may remove it from their profile.

Figure 4 more precisely illustrates steps S4 to S7.

At step S21, the CNN of the artificial intelligence module separates the health document into a class comprising a text class, including the prescription and the biological examination result.

At step S22, the artificial intelligence module implements a logistic regression model. The logistic regression model is applied to the classified text health document to classify them into a prescription class (step S23) and a biological examination result class (step S24).

At step S21, the CNN of the artificial intelligence module also separates the health document into an X-ray image class (step S25) and a photography of a body part class (step S26). For the X-ray images and the photography of a body part, the method directly goes to step S35.

At step S27, the optical character recognition (OCR) technique of the artificial intelligence module is applied to the classified prescription and biological examination result health document. Such optical recognition technique are well-known in the art and won't be further described. The optical character recognition technique returns a file containing the text contained in the classified prescription and biological examination result health document as well as its geometric-related information in sentence level and word level.

A further step of text pre-processing may be applied after step S27. This further step may be applied for health document which has been sent will poor quality. A data cleaning may be applied to the extracted text by removing accents, transforming words to lowercase, unifying the format of numbers and removing space between them, removing the stop words and removing sentences with one word including less than two characters.

This step allows to obtain extracted text where all the mistakes that the OCR could have been made during its extraction are removed.

At step S28, the artificial intelligence module implements, as a machine learning model, a named entity recognition (NER), which is an application of Natural Language Processing (NLP). The NER is applied on the classified prescription health document. Before extracting the useful sentences from the text of the prescription health document, a categorization of the text sentences containing drug is applied. It has the advantage of increasing the efficiency of the NER model since it has made the model focus only on sentences that contain the useful sentences.

At step S30, the drug contained in the prescription document are recognized and classified based on the drug names store din the second data storage system. Moreover, the model has been trained with sentences describing how a drug should be taken, i.e. the posology. The posology is then also classified at step S31.

A bidirectional Long-short-Term Memory Architecture is used for steps S30 and S31.

If the drug and posology are not recognized 100% by the LSTM, they are not taken into account to avoid any error.

At step S32, the extracted drug and posology are matched together. To do so, the geometric-related information obtained at step S27 is used. More precisely, a drug is associated to a posology when the distance between them is inferior to a predetermined threshold. If a plurality of sentences is spaced from a distance inferior to the threshold, it is considered that they belong to a same section and are all associated together. Also, when a plurality of sentences is aligned horizontally, it is considered that they belong to a same section and are all associated together.

Concerning the biological examination result, a table extraction is performed at step S33. To do so, the artificial intelligence module implement a plurality of matching models to extract parameters in the biological examination result health document. Each matching model is designed to detect a specific parameter with its associated value and unit.

Finally, step S34 corresponds to step S10 of figure 3.

Advantageously, the structuration and extraction of data described with reference to figure 4 are performed in real time.

It results from above that the system and method according to the present application allows to have a fully automated and autonomous health platform.

While exemplary embodiment of the invention has been described with reference to two main embodiments, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

## Claims

1. An automated system (1) for managing an artificial intelligence health platform comprising:
➢ a first data storage system (11) storing
∘ a plurality of user identification elements, each user identification element being associated with a user among a plurality of users,
∘ for each user, at least one health document linked to said user identification element,
➢ a server (10) configured to communicate with said first data storage system (11), said server being configured to:
∘ receive a message with an attached file, said attached file being an attached health document,
∘ implement an artificial intelligence module (101) implementing a machine learning model, said machine learning model being configured to perform optical recognition and data extraction in the attached health document, said extracted data being a user identification element,
∘ record and link said health document to the corresponding user in the first data storage system (11) on the basis of the extracted personal identification element.

2. System (1) according to claim 1, further comprising a user terminal (13) comprising a user interface (130), said user terminal (13) being configured to communicate with the server (10), wherein the user terminal (13) is configured to allow a user to visualize their health document linked to their user personal identification element through the user interface.

3. System (1) according to claims 1 or 2, wherein following the recording and linking of said health document to the corresponding user in the first data storage system (11), the server (10) is further configured to send a notification to the corresponding user, said notification indicating that a health document has been linked and recorded.

4. System (1) according to claim 3, wherein the notification is sent to the user terminal (13), and/or to a phone number of the user and/or to an email address of the user.

5. System (1) according to claims 3 or 4, wherein upon the receiving of the notification, the user interface (130) is configured to allow the user to confirm to the server (10) whether the recorded and linked health document has been linked and recorded to the correct user.

6. System (1) according to any of claims 1 to 5, wherein the machine learning model of the artificial intelligence module (101) is further configured to classify the attached health data document into one of the following classes of document:
➢ text document,
➢ image document such as X-Ray imaging, echography,
➢ photography document of a part of a body.

7. System (1) according to any of claims 1 to 6, further comprising a second data storage (12) system in communication with said server (10), said second data storage system (12) storing at least drugs names and diseases names, said second data storage system (12) being automatically and continuously constructed by means of the machine learning model of the artificial intelligence module (101), wherein said machine learning model is configured to scan at least one existing official database regrouping drug names and diseases name, to store said drug names and diseases in the second data storage system (12) and to associate said drug names and disease names with one single identifier in the second data storage system (12).

8. System (1) according to the preceding claim, wherein said machine learning model of the artificial intelligence module (101) is further configured to regularly update said second data storage system (12) with newly added drug names and disease names identified in the official database.

9. System (1) according to any of claims 1 to 8, wherein the machine learning model of the artificial intelligence module (101) is further configured to classify sentences from the documents classified in the text document class between useful sentences and useless sentences, the machine learning model being further configured to attach said useful sentences to one single identifier of the second data storage system (12).

10. System (1) according to any of claims 1 to 9, wherein the attached document is a digital document in one of the following formats:
➢ Pdf, or
➢ Image format.

11. System (1) according to any of claims 1 to 10, wherein the user identification element comprises:
➢ a name and/or a forename of said user,
➢ an insurance number, and/or
➢ a personal code linked to their profile.

12. An automated method for managing an artificial intelligence health platform managing system comprising:
➢ storing in a first data storage system (11):
∘ a plurality of user identification elements, each user identification element being associated with a user among a plurality of users,
∘ for each user, at least one health document linked to said user identification element,
➢ providing a communication between a server (10) and said first data storage system (11), wherein said server (10):
∘ receives a message with an attached file, said attached file being an attached health document,
∘ implements an artificial intelligence module (101) implementing a machine learning model performing optical recognition data extraction in the attached health document, said extracted data being a user personal identification element,
∘ records and links said health document to the corresponding user in the data storage system on the basis of the extracted user identification element.
